# EUROPEAN PATENT APPLICATION

(11) **EP 3 456 314 A1**
(43) Date of publication of application: **20.03.2019**
(21) Application number: 17191107.6
(22) Date of filing: 14.09.2017
(51) Int. Cl.: A61K 9/00, A61K 47/06, A61K 47/10, A61K 47/12, A61K 47/14, A61K 47/20, A61K 47/22, A61K 9/06, A61K 9/107, A61K 31/57

(54) **TOPICAL FORMULATION COMPRISING 17-KETOLIC CORTICOSTEROID**

(71) Applicant: Tiofarma B.V., 3261 LW Oud-Beijerland (NL)
(72) Inventor: VAN HEUGTEN, Anton Joris Pancras, 3011 ZW Rotterdam (NL)
(74) Representative: Nederlandsch Octrooibureau

(57) **Abstract**

The invention relates to a topical formulation comprising:
a) 0.01-3 wt.% of one or more 17-ketolic corticosteroids comprising a ketolic residue in the 17-position that is represented by the following formula (I):

-COCH₂OH (I) ;

b) 1-50 wt.% of polar solvent selected from propylene glycol, glycerol, ethanol, sorbitol, polyethylene glycol and combinations thereof;
c) 0.01-1 wt.% of sulfite compound selected from sodium metabisulfite, potassium metabisulfite, sodium bisulfite, potassium bisulfite, sodium sulfite and combinations thereof;
d) 40-98.9 wt.% of apolar substance selected petrolatum, wax, mineral oil, fat, fatty alcohols and combinations thereof;
e) 0-5 wt.% water;
wherein the composition is an emulsion comprising a continuous apolar phase containing the apolar substance and a dispersed polar phase containing the polar solvent; and wherein the combination of components a) to e) constitutes at least 80 wt.% of the pharmaceutical composition.

The 17-ketolic corticosteroid in the topical formulations of the present invention is highly stable. Even when the formulation is exposed to atmospheric conditions for several weeks no more than limited decomposition of the 17-ketolic corticosteroid is observed.

## Description

### TECHNICAL FIELD OF THE INVENTION

The present invention relates to a topical formulation comprising a 17-ketolic corticosteroid. The topical formulation of the present invention offers the advantage that the 17-ketolic corticosteroid is effectively protected against oxidative degradation.

The invention also relates to the use of the aforementioned topical formulation in the treatment of skin disorders.

### BACKGROUND OF THE INVENTION

Corticosteroids are a class of steroid hormones that are produced in the adrenal cortex of vertebrates, or synthetic analogues of these hormones that have been developed by the pharmaceutical industry. The other main classes of steroid hormones are progestogens, androgens and estrogens.

Corticosteroids are involved in a wide range of physiologic processes, including stress response, immune response, and regulation of inflammation, carbohydrate metabolism, protein catabolism, blood electrolyte levels, and behavior.

Like all steroids, corticosteroids comprise a core structure that is composed of seventeen carbon atoms, bonded in 4 "fused" rings: three six-member cyclohexane rings and one five-member cyclopentane ring. Steroids vary by the functional groups attached to this four-ring core and by the oxidation state of the rings.

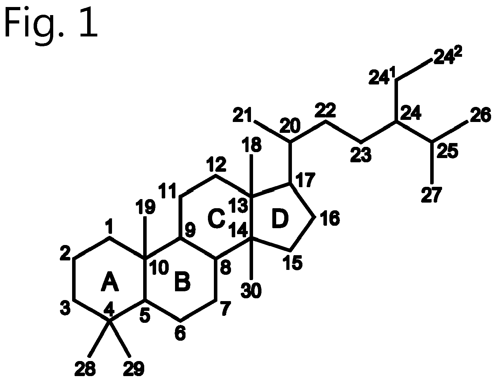

The core structure of 4 fused rings contains 17 carbon atoms, and the numbering assigned to the carbon atoms within this structure as well as to carbon atoms attached to this structure are shown in Figure 1.

Within the class of corticosteroids there is a sub-group of corticosteroids (17-ketolic corticosteroids) that contains a 21-hydroxyacetone sidechain in the 17-position. Examples of such 17-ketolic corticosteroids include hydrocortisone, methylprednisolone, prednisolone, prednisone, triamcinolone, budesonide, triamcinolone acetonide, beclomethasone, bethamethasone, dexamethasone, bethametasone valerate, hydrocortisone butyrate, hydrocortisone valerate and desoximetason.

It is known that the 17-hydroxyacetone sidechain of 17-ketolic corticosteroids can easily undergo oxidative and/or hydrolytic degradation. The associated lack of stability of 17-ketolic corticosteroids poses a particular challenge for the application of these corticosteroids in topical formulations. This is because, after opening of the sealed package, topical formulations are typically used over a period of several weeks or even months. During this period the formulation is exposed to atmospheric oxygen and moisture, i.e. to conditions that promote degradation of the 17-ketolic corticosteroids.

Jackson et al. (Hemiacetal formation by some 17-ketolic corticosteroids, Proc. Analyt. Div. Chem. Soc, 12 (1975), 296-299) report that pre-formulation studies on the development of an aqueous propylene glycol gel formulation revealed that the 17-ketolic sidechain of 17-ketolic corticosteroids could react with propylene glycol to form a 21-hemiacetal derivative as a decomposition product. The authors observe that the major factor involved was found to be oxidation of the steroid by dissolved oxygen, especially in the presence of trace metal ions such as iron (III). The authors hypothesize that the extent of hemiacetal formation could be controlled by the addition of chelating agents such as EDTA and citric acid or by the addition of sodium metabisulphite as antioxidant. The authors further refer to US 3,856,954 which mentions a stabilizing influence exerted by a combination of sodium metabisulphite and citric acid.

US 3,856,954 describes aqueous gel compositions comprising, by weight, 0.01 to 1% of a corticosteroid, 0.5 to 2% of a carboxymethylene hydrocolloid polymer of acrylic acid cross linked with polyallyl sucrose, 10 to 20% of propylene glycol, 10 to 20% of isopropyl alcohol or ethyl alcohol and 0.01 to 0.15% of alkali metal bisulfite. The examples describe aqueous gel compositions containing triamcinolone acetonide. It is observed in the US patent that since there is sometimes some interaction of the ingredients or deleterious factors introduced during processing, packaging or storing of the compositions, it is desirable to include in the compositions a small, but effective amount of alkali metal metabisulfite and/or citric acid.

WO 96/40042 describes a pharmaceutical composition comprising a therapeutically effective amount of triamcinolone acetonide in admixture with an aqueous pharmaceutical acceptable carrier providing said composition with properties resistant to triamcinolone acetonide degradation in the presence of contaminants such as copper ions. The pharmaceutical composition preferably contains a metal sequestering agents such as EDTA.

US 2016/0067266 describes a storage stable ointment comprising:
- a vitamin D compound;
- a corticosteroid; and
- an N,N-di(C₁-C₈) alkylamino substituted, (C₁-C₁₈) alkyl (C₂-C₁₈)carboxylic ester.
The examples describe a composition comprising calcipotriene (0.005%), betamethasone dipropionate (0.064%), docecyl-2-(N,N-dimethylamino)-propionate (1%), vitamin E (0.002%), mineral oil (3%) and white petrolatum (q.s. 100).

### SUMMARY OF THE INVENTION

The inventors have developed a topical formulation comprising 17-ketolic corticosteroid that is exceptionally stable. This topical formulation comprises:
a) 0.01-3 wt.% of one or more 17-ketolic corticosteroids comprising a ketolic residue in the 17-position that is represented by the following formula (I):

   -COCH₂OH (I);
b) 1-50 wt.% of polar solvent selected from propylene glycol, glycerol, ethanol, sorbitol, polyethylene glycol and combinations thereof;
c) 0.01-1 wt.% of sulfite compound selected from sodium metabisulfite, potassium metabisulfite, sodium bisulfite, potassium bisulfite, sodium sulfite and combinations thereof;
d) 40-98.9 wt.% of apolar substance selected petrolatum, wax, mineral oil, fat, fatty alcohols and combinations thereof;
e) 0-5 wt.% water;
wherein the composition is an emulsion comprising a continuous apolar phase containing the apolar substance and a dispersed polar phase containing the polar solvent; and wherein the combination of components a) to e) constitutes at least 80 wt.% of the pharmaceutical composition.

The 17-ketolic corticosteroid in the topical formulations of the present invention is highly stable. Even when the formulation is exposed to atmospheric conditions for several weeks, no more than limited decomposition of the 17-ketolic corticosteroid is observed.

### DETAILED DESCRIPTION OF THE INVENTION

A first aspect of the invention relates to a pharmaceutical composition suitable for topical application, said composition comprising:
a) 0.01-3 wt.% of one or more 17-ketolic corticosteroids comprising a ketolic residue in the 17-position that is represented by the following formula (I):

   -COCH₂OH (I);
b) 1-50 wt.% of polar solvent selected from propylene glycol, glycerol, ethanol, sorbitol, polyethylene glycol and combinations thereof;
c) 0.01-1 wt.% of sulfite compound selected from sodium metabisulfite, potassium metabisulfite, sodium bisulfite, potassium bisulfite, sodium sulfite and combinations thereof;
d) 40-98.9 wt.% of apolar substance selected petrolatum, wax, mineral oil, fat, fatty alcohols and combinations thereof;
e) 0-5 wt.% water;
wherein the composition is an emulsion comprising a continuous apolar phase containing the apolar substance and a dispersed polar phase containing the polar solvent; and wherein the combination of components a) to e) constitutes at least 80 wt.% of the pharmaceutical composition.

The term "petrolatum" as used herein refers to is a semi-solid mixture of saturated hydrocarbons with carbon numbers mainly higher than 25 (CAS number 8009-03-8). Synonyms for petrolatum are "petroleum jelly" and "vaseline".,

The term "wax" as used herein refers to animal waxes, vegetable waxes, mineral waxes and paraffin waxes. Examples of animal waxes include beeswax and lanolin (wool wax). Examples of vegetable waxes include candelilla wax, carnauba wax, castor wax (hydrogenated castor oil), rice bran wax and soy wax. Examples of mineral waxes include ceresin and ozocerite.

The term "mineral oil" is a synonym for "paraffin oil" and as used herein refers to liquid mixture of higher alkanes from a mineral source, e.g. a distillate of petroleum.

The term "fat" as used herein refers to glyceride esters selected from the group of triglcyrides, diglyerides and combinations thereof.

The term "fatty alcohol" as used herein refers to a straight-chain primary alcohol comprising at least 12 carbon atoms.

Examples of pharmaceutical compositions suitable for topical application include liquids and semi-solid compositions. Preferably, the pharmaceutical composition of the present invention is a semi-solid composition, such as an ointment.

The combination of components a) to e) preferably constitutes at least 90 wt.%, more preferably at least 95 wt.% and most preferably at least 98 wt.% of the present pharmaceutical composition.

The pharmaceutical composition preferably contains at least 0.03 wt.%, more preferably at least 0.05 wt.% and most preferably at least 0.08 wt.% of the one or more 17-ketolic corticosteroids.

Calculated by weight of the polar solvent, the composition typically contains the one or more 17-ketolic corticosteroids in a concentration of at least 0.1 wt.%, more preferably at least 0.3 wt.% and most preferably at least 0.5 wt.%. The concentration of the one or more 17-ketolic corticosteroids preferably does not exceed the maximum solubility of the corticosteroid(s) in the polar solvent.

In a preferred embodiment, the one or more 17-ketolic corticosteroids employed in accordance with the present invention comprise a 17-hydroxyl residue that is optionally esterified, e.g. with an organic acid selected from propionic acid, butanoic acid and pentanoic (=valeric) acid.

According to a particularly preferred embodiment, the 17-ketolic corticosteroid comprises a 17-hydroxyl residue and a 16-hydroxyl residue that together form a cyclic ketal with acetone (acetonide).

The one or more 17-ketolic corticosteroids that are contained in the pharmaceutical composition are preferably selected from hydrocortisone, methylprednisolone, prednisolone, prednisone, triamcinolone, budesonide, triamcinolone acetonide, beclomethasone, bethamethasone, dexamethasone, bethametasone valerate, hydrocortisone butyrate, hydrocortisone valerate and desoximetason.

According to a particularly preferred embodiment the pharmaceutical composition contains at least 0.01 wt.%, more preferably at least 0.03 wt.%, even more preferably at least 0.05 wt.% and most preferably at least 0.08 wt.% of triamcinolone acetonide.

The polar solvent is preferably contained in the pharmaceutical composition in a concentration of 2-40 wt.%, more preferably of 3-30 wt.% and most preferably of 4-20 wt.%.

In accordance with another preferred embodiment at least 50 wt.%, more preferably at least 80 wt.% and most preferably at least 90 wt.% of the polar solvent is propylene glycol.

The pharmaceutical composition of the present invention preferably contains at least 0.02 wt.%, more preferably at least 0.03 wt.% and most preferably at least 0.04 wt.% of the sulfite compound. Typically, the composition does not contain more than 2 wt.%, more preferably not more than 1 wt.% of the sulfite compound.

According to a particularly preferred embodiment, the pharmaceutical composition contains at least 0.01 wt.%, more preferably at least 0.02 wt.% and most preferably at least 0.04 wt.% of sulfite compound selected from sodium metabisulfite, potassium metabisulfite and combinations thereof.

Most preferably, the composition contains at least 0.01 wt.%, more preferably at least 0.02 wt.% and most preferably at least 0.04 wt.% of sodium metabisulfite.

The sulfite compound and the one or more 17-ketolic corticosteroids are typically contained in the pharmaceutical composition in a molar ratio (sulfite:corticosteroid) within the range of 1:3 to 20:1, more preferably within the range of 1:2 to 15:1 and most preferably within the range of 1:1 to 10:1.

The apolar substance is preferably contained in the pharmaceutical composition in a concentration of 60-97 wt.%, more preferably of 70-96 wt.% and most preferably of 75-95 wt.%.

According to another preferred embodiment, the apolar substance contains at least 60 wt.%, more preferably at least 70 wt.% and most preferably at least 75 wt.% petrolatum.

The water content of the pharmaceutical composition preferably does not exceed 5 wt.%, more preferably it does not exceed 3 wt.%.

The stability of the pharmaceutical composition may be further improved by including a metal chelant. Preferably, the composition contains at least 0.01 wt.%, more preferably at least 0.05 wt.% and most preferably 0.1 - 2 wt.% of a metal chelant selected from 1,10-phenanthroline, ethylenediaminetetraacetate (EDTA), citric acid, maleic acid, fumaric acid and combinations thereof.

According to a particularly preferred embodiment, the pharmaceutical composition contains at least 0.01 wt.%, more preferably at least 0.05 wt.% and most preferably at least 0.1 wt.% of a metal chelant selected from 1,10-phenanthroline, citric acid, maleic acid, fumaric acid and combinations thereof EDTA.

Other ingredients that may suitably be incorporated in the pharmaceutical composition of the present invention include penetration enhancers, emollients, emulsifiers, anti-oxidants, preservatives, perfumes and pigments.

In accordance with an advantageous embodiment of the present invention the pharmaceutical composition is a semi-solid emulsion comprising 60-97 wt.% of the continuous apolar phase containing the apolar substance; and 1.1-40 wt.% of the dispersed polar phase containing the polar solvent. More preferably, the semi-solid emulsion comprises 70-95 wt.% of the continuous apolar phase and 5-30 wt.% of the dispersed polar phase.

The continuous apolar phase and the dispersed polar phase together preferably constitute at least 95 wt.%, more preferably at least 98 wt.% of the semi-solid emulsion.

The apolar substance typically constitutes at least 90 wt.%, more preferably at least 95 wt.% of the continuous apolar phase of the semi-solid emulsion.

According to another preferred embodiment, the continuous apolar phase of the emulsion contains at least 50 wt.%, more preferably at least 70 wt.% and most preferably at least 75 wt.% petrolatum.

The polar solvent preferably constitutes at least 80 wt.%, more preferably at least 85 wt.% of the dispersed polar phase the semi-solid emulsion.

The dispersed polar phase of the emulsion preferably contains at least 50 wt.%, more preferably at least 80 wt.% and most preferably at least 90 wt.% of propylene glycol.

The dispersed polar phase of the semi-solid emulsion preferably has a mean volume weighted average diameter in the range of 5 to 200 µm, more preferably in the range of 10 to 100 µm.

Another aspect of the invention relates to the use of a pharmaceutical composition as defined herein before in the treatment of a skin disorder, said use comprising topical application of the pharmaceutical composition.

Examples of skin disorders that may be treated with the pharmaceutical composition include psoriasis, dermatitis, eczema, vitiligo, phimosis and lichen sclerosus.

The invention is further illustrated by the following non-limiting examples.

### EXAMPLES

### Example 1

Ointments containing 0.1 % (w/w) triamcinolone acetonide were prepared on the basis of the recipes shown in Table 1.

**Table 1**

| | **Wt.%** | | | |
|---|---|---|---|---|
| | **1** | **2** | **A** | **B** |
| Triamcinolone acetonide | 0.1 | 0.1 | 0.1 | 0.1 |
| Sodium metabisulfite | 0.1 | 0.1 | | |
| 1,10-Phenanthroline | | 0.1 | | 0.1 |
| Propylene glycol | 10.0 | 10.0 | 10.0 | 10.0 |
| Lanolin | 10.0 | 10.0 | 10.0 | 10.0 |
| Petrolatum | 79.8 | 79.7 | 79.9 | 79.8 |

The stability of these ointments was investigated by storing the ointments at 60 °C and measuring the degradation products using HPLC-UV. The method was based on the "related substances" method described in the Pharmacopoeia monograph for triamcinolone acetonide (monograph number 0533).

The results of the stability study are summarized in Table 2.

**Table 2**

| **Storage time (days)** | **% triamcinolone acetonide** | | | |
|---|---|---|---|---|
| | **1.1** | **1.2** | **1.A** | **1.B** |
| 0 | 99.9 | 99.9 | 99.8 | 99.9 |
| 28 | 99.4 | 99.2 | 87.5 | 99.2 |
| 84 | 97.8 | 98.1 | 84.2 | 82.9 |
| 159 | 90.4 | 94.8 | 74.7 | 75.4 |

The results indicate that the stability of triamcinolone acetonide is improved by the addition of sodium metabisulfite and that adding a sequestering agent such as 1,10-phenantroline further improves the stability. 1,10-phenantroline by itself is not able to increase triamcinolone acetonide stability.

### Example 2

Ointments containing 0.1% (w/w) triamcinolone acetonide are prepared on the basis of the recipes shown in Table 3.

**Table 3**

| | **Wt.%** | |
|---|---|---|
| | **2.1** | **2.2** |
| Triamcinolone acetonide | 0.1 | 0.1 |
| Sodium metabisulfite | 0.1 | 0.1 |
| Citric acid | 0.1 | 0 |
| Propylene glycol | 10.0 | 10.0 |
| Lanolin | 10.0 | 10.0 |
| Petrolatum | 79.7 | 79.8 |

The stability of these ointments is tested in the same way as in Example 1. The tests show that ointment 2.1 is more stable than 2.2. The improved stability of ointment 2.1 is believed to be associated with the metal sequestering effect of citric acid that negates the destabilizing effect of traces of metal cations such as Cu²⁺.

### Example 3

Ointments containing 0.1 % (w/w) triamcinolone acetonide were prepared on the basis of the recipes shown in Table 4.

**Table 4**

| | **Wt.%** | |
|---|---|---|
| | **3.1** | **3.2** |
| Triamcinolone acetonide | 0.1 | 0.1 |
| Sodium metabisulfite | 0.1 | 0.1 |
| Fumaric acid | 0.1 | 0 |
| Propylene glycol | 10.0 | 10.0 |
| Lanolin | 10.0 | 10.0 |
| Petrolatum | 79.7 | 79.8 |

The stability of these ointments is tested in the same way as in Example 1. The tests show that ointment 3.1 is more stable than 3.2. The improved stability of ointment 3.1 is believed to be associated with the metal sequestering effect of fumaric acid that negates the destabilizing effect of traces of metal cations such as Cu²⁺.

### Example 4

Ointments containing 0.1 % (w/w) triamcinolone acetonide were prepared on the basis of the recipes shown in Table 3.

**Table 5**

| | **Wt.%** | |
|---|---|---|
| | **4.1** | **4.2** |
| Triamcinolone acetonide | 0.1 | 0.1 |
| Sodium metabisulfite | 0.1 | 0.1 |
| Maleic acid | 0.1 | 0 |
| Propylene glycol | 10.0 | 10.0 |
| Lanolin | 10.0 | 10.0 |
| Petrolatum | 79.7 | 79.8 |

The stability of these ointments is tested in the same way as in Example 1. The tests show that ointment 4.1 is more stable than 4.2. The improved stability of ointment 4.1 is believed to be associated with the metal sequestering effect of maleic acid that negates the destabilizing action of traces of metal cations such as Cu²⁺.

## Claims

1. A pharmaceutical composition suitable for topical application, said composition comprising:
a) 0.01-3 wt.% of one or more 17-ketolic corticosteroids comprising a ketolic residue in the 17-position that is represented by the following formula (I):
-COCH₂OH (I);
b) 1-50 wt.% of polar solvent selected from propylene glycol, glycerol, ethanol, sorbitol, polyethylene glycol and combinations thereof;
c) 0.01-1 wt.% of sulfite compound selected from sodium metabisulfite, potassium metabisulfite, sodium bisulfite, potassium bisulfite, sodium sulfite and combinations thereof;
d) 40-98.9 wt.% of apolar substance selected petrolatum, wax, mineral oil, fat, fatty alcohols and combinations thereof;
e) 0-5 wt.% water;
wherein the composition is an emulsion comprising a continuous apolar phase containing the apolar substance and a dispersed polar phase containing the polar solvent; and wherein the combination of components a) to e) constitutes at least 80 wt.% of the pharmaceutical composition.

2. Pharmaceutical composition according to claim 1, wherein the composition contains the one or more 17-ketolic corticosteroids in a concentration of at least 0.1 % by weight of the polar solvent.

3. Pharmaceutical composition according to claim 1 or 2, wherein at least 50 wt.% of the polar solvent is propylene glycol.

4. Pharmaceutical composition according to any one of the preceding claims, wherein the one or more 17-ketolic corticosteroids are selected from hydrocortisone, methylprednisolone, prednisolone, prednisone, triamcinolone, budesonide, triamcinolone acetonide, beclomethasone, bethamethasone, dexamethasone, bethametasone valerate, hydrocortisone butyrate, hydrocortisone valerate and desoximetason.

5. Pharmaceutical composition according to any one of the preceding claims, wherein x is 2.

6. Pharmaceutical composition according to any one of the preceding claims, wherein R represents H.

7. Pharmaceutical composition according to any one of the preceding claims, wherein the pharmaceutical composition contains at least 0.01 wt.% of triamcinolone acetonide.

8. Pharmaceutical composition according to any one of the preceding claims, wherein the pharmaceutical composition contains at least 0.1 wt.% of sulfite compound selected from sodium metabisulfite, potassium metabisulfite and combinations thereof.

9. Pharmaceutical composition according to any one of the preceding claims, wherein the composition contains at least 0.01 wt.% of a metal chelant selected from 1,10-phenanthroline, ethylenediaminetetraacetate (EDTA), citric acid, maleic acid, fumaric acid and combinations thereof.

10. Pharmaceutical composition according to any one of the preceding claims, wherein the composition is a semi-solid emulsion comprising 60-97 wt.% of the continuous apolar phase; and 1.1-40 wt.% of a dispersed polar phase.

11. Pharmaceutical composition according to any one of the preceding claims, wherein the continuous apolar phase and the dispersed polar phase together constitute at least 95 wt.% of the pharmaceutical composition.

12. Pharmaceutical composition according to any one of the preceding claims, wherein the apolar substance constitutes at least 90 wt.% of the continuous apolar phase.

13. Pharmaceutical composition according to any one of the preceding claims, wherein the polar solvent constitutes at least 80 wt.% of the dispersed polar phase.

14. Pharmaceutical composition according to any one of the preceding claims, wherein the dispersed polar phase has a mean volume weighted average diameter in the range of 5 to 200 µm.

15. Pharmaceutical composition for use in the treatment of a skin disorder, said use comprising topical application of a pharmaceutical composition according to any one of the preceding claims.
